# EUROPEAN PATENT SPECIFICATION

(11) **EP 4 238 565 B1**
(45) Date of publication and mention of the grant of the patent: **24.09.2025**
(21) Application number: 21885567.4
(22) Date of filing: 19.02.2021
(51) Int. Cl.: A61K 9/00, A61K 31/4545, A61K 9/20, A61P 15/02, A61P 31/22, A61P 35/00

(54) **PHARMACEUTICAL COMPOSITION FOR PREVENTING OR TREATING VIRAL PERIVAGINAL DISEASE**
PHARMAZEUTISCHE ZUSAMMENSETZUNG ZUR PRÄVENTION ODER BEHANDLUNG VON VIRUSPERIVAGINALEN ERKRANKUNGEN
COMPOSITION PHARMACEUTIQUE POUR LA PRÉVENTION OU LE TRAITEMENT D'UNE MALADIE PÉRIVAGINALE VIRALE

(30) Priority: 28.10.2020 JP 2020180339
(43) Date of publication of application: 06.09.2023
(73) Proprietor: KinoPharma, Inc., Chuo-ku, Tokyo 103-0023 (JP)
(72) Inventor: ONOGI Hiroshi, Tokyo 103-0023 (JP); YAMAGUCHI Tetsuo, Tokyo 103-0023 (JP); SATO Katsuhiko, Tokyo 103-0023 (JP)
(74) Representative: Hoffmann Eitle
(86) International application number: PCT/JP2021/006418
(87) International publication number: WO 2022/091442

(56) References cited:
- WO-A1-2009/020198
- WO-A1-2020/218517
- WO-A1-2020/218518
- JP-A- S63 275 519
- SUMI ERIKO ET AL: "Safety and Plasma Concentrations of a Cyclin-dependent Kinase 9 (CDK9) Inhibitor, FIT039, Administered by a Single Adhesive Skin Patch Applied on Normal Skin and Cutaneous Warts", CLINICAL DRUG INVESTIGATION, vol. 39, no. 1, 4 October 2018 (2018-10-04), NZ, pages 55 - 61, XP093155716, ISSN: 1173-2563, DOI: 10.1007/s40261-018-0712-7
- MAKOTO YAMAMOTO ET AL: "CDK9 inhibitor FIT-039 prevents replication of multiple DNA viruses", THE JOURNAL OF CLINICAL INVESTIGATION, vol. 124, no. 8, 8 July 2014 (2014-07-08), pages 3479 - 3488, XP055762900, DOI: 10.1172/JCI73805
- ANWAR AZLINDA ET AL: "The Kinase Inhibitor SFV785 Dislocates Dengue Virus Envelope Protein from the Replication Complex and Blocks Virus Assembly", PLOS ONE (2011), 6(8), E23246, 17 August 2011 (2011-08-17), pages 1 - 8, XP093197324, Retrieved from the Internet <URL:http://www.plosone.org/article/fetchObjectAttachment.action?uri=info:doi/10.1371/journal.pone.0023246&representation=PDF> DOI: 10.1371/journal.pone.0023246
- AJIRO MASAHIKO, SAKAI HIROYUKI, ONOGI HIROSHI, YAMAMOTO MAKOTO, SUMI ERIKO, SAWADA TERUO, NOMURA TAKASHI, KABASHIMA KENJI, HOSOYA : "CDK9 Inhibitor FIT-039 Suppresses Viral Oncogenes E6 and E7 and Has a Therapeutic Effect on HPV-Induced Neoplasia", CLINICAL CANCER RESEARCH, ASSOCIATION FOR CANCER RESEARCH, US, vol. 24, no. 18, 15 September 2018 (2018-09-15), US, pages 4518 - 4528, XP055938710, ISSN: 1078-0432, DOI: 10.1158/1078-0432.CCR-17-3119
- OKAMOTO MIKA, HIDAKA AKEMI, TOYAMA MASAAKI, HOSOYA TAKAMITSU, YAMAMOTO MAKOTO, HAGIWARA MASATOSHI, BABA MASANORI: "Selective inhibition of HIV-1 replication by the CDK9 inhibitor FIT-039", ANTIVIRAL RESEARCH, ELSEVIER BV, NL, vol. 123, 1 November 2015 (2015-11-01), NL , pages 1 - 4, XP055938712, ISSN: 0166-3542, DOI: 10.1016/j.antiviral.2015.08.012

## Description

### [Technical Field]

The present invention relates to a pharmaceutical composition for use in the prevention and/or treatment of viral perivaginal diseases.

### [Background Art]

Cervical cancer is one of the most common malignant lesions in women, and its treatment and early detection are desired worldwide. Cervical cancer accounts for about 70% of uterine cancers and about 20% of all cancers in obstetrics and gynecology, and is an important disease target. Cervical cancer affects 500,000 people worldwide and about 10,000 people in Japan every year, and the number of patients is increasing.

It is known that cervical cancer is mostly caused by human papillomavirus (HPV) infection. Among 100 types of HPV, mainly 14 types (16, 18, 31, 33, 35, 39, 45, 51, 52, 56, 58, 59, 66 and 68) are called high-risk types, especially HPV16 and 18 are known to have the highest relationship with the induction of cervical cancer.

HPV is a common virus that infects both men and women, and is often sexually transmitted. However, even when infected with high-risk types of HPV, in many cases (about 90%), the infection disappears spontaneously due to the host's immunity. On the other hand, HPV infection persists in about 10% of people, and some of those who do not recover spontaneously develop to cervical cancer over a period of several to 10 years through a precancerous lesion called dysplasia.

Cervical dysplasia is the precursor to cervical cancer (precancerous lesion) and is also called cervical intraepithelial neoplasia (CIN). In recent years, cervical cancer and cervical dysplasia are rapidly increasing among women in their 20s and 30s in Japan. Cervical dysplasia is classified into three types according to the severity of the lesion: low-grade dysplasia (CIN1), moderate dysplasia (CIN2), and high-grade dysplasia/carcinoma in situ (CIN3). In addition, cervical dysplasia is classified into LSIL (mild abnormality) and HSIL (moderate to severe abnormality) according to the Bethesda classification based on vaginal cytology. Squamous epithelial lesions of the cervix are known to progress stepwise, with low-grade dysplasia, moderate dysplasia, high-grade dysplasia/carcinoma in situ, microinvasive squamous epithelial carcinoma, and invasive carcinoma. Cervical dysplasia often shows no subjective symptoms, and is often discovered during cervical cancer screening (cytological examination), making early treatment important. In addition, there are reports that the detection rate of high-risk types of HPV is about 60% for low-grade dysplasia, about 85% for moderate dysplasia, about 90% for high-grade dysplasia/carcinoma in situ in squamous epithelium, and about 100% for squamous cell carcinoma.

Diagnosis of cervical dysplasia is mainly performed by methods called cytology, colposcopy, and histology. Cytological examination is the primary examination in cervical cancer screening, and if abnormalities are found in the cytological examination, colposcopy examination and histological examination are performed as secondary examination (detailed examination). Current diagnostics often involve observation rather than immediate treatment in cases of low-grade dysplasia (CIN1) and moderate dysplasia (CIN2). The reason for this is that about half of the patients tend to spontaneously heal (disappear) without treatment, while current surgical treatments are very invasive and burdensome to the patients.

Surgery is the treatment of choice for high-grade dysplasia/carcinoma in situ (CIN3) and long-term persistence of CIN2. A typical surgical treatment includes cervical conization, in which the cervix (the entrance portion) is excised in a cone shape. This surgery may be performed not only for CIN3, but also for microinvasive squamous epithelial carcinoma (stage Ia1). In particular, this surgery is the first choice for patients who wish to become pregnant (uterine preservation), but total hysterectomy may be selected for patients who do not require uterine preservation.

In addition, there are cases in which cytological examination suggests moderate/severe dysplasia to carcinoma in situ or higher, but it is difficult to make a definitive diagnosis by colposcopy or histology, and especially for elderly patients, it is often the case that invasive cancer is suspected by cytological examination, but the existence of a lesion is unknown by tissue examination under colposcopy or endocervical curettage. In such cases, cervical conization is required for diagnostic purposes.

Currently, there is no effective drug therapy in the treatment of cervical dysplasia/intraepithelial neoplasia. On the other hand, although HPV vaccines are effective in preventing HPV infection, they are not effective in treating dysplasia or cancer. Therefore, therapeutic agents for cervical dysplasia and intraepithelial neoplasia are desired.

Condyloma acuminata is one of the sexually transmitted diseases caused by human papillomavirus, infecting both men and women, and it is reported that several dozen people per 100,000 people are infected with the disease in Japan. In women, it affects the vagina, vaginal vestibule, labia major and minor, cervix, anus, and periphery thereof. Even if it develops, it often heals spontaneously. Human papillomaviruses that cause condyloma acuminata are mainly HPV6 and 11, and these are types of viruses with low carcinogenic risk. However, they can also be co-infected with malignant types of viruses such as HPV 16 and 18, which have a high risk of developing cervical cancer. In women, when condyloma acuminata develops in the vulva, they may be infected with malignant HPV that causes uterine cancer.

Genital herpes is infected with the herpes simplex virus and develops in or around the genitals. In women, lesions are seen in the vulva, vagina and cervix. Genital herpes may be an initial infection by a virus that has entered from the outside, or it may be a reactivation of a latent virus in the sacral marrow ganglion.

It is known that aniline derivatives, including the CDK9 inhibitor N-[5-fluoro-2-(1-piperidinyl)phenyl] isonicotinthioamide, can be used as antiviral agents to suppress virus production (For example, Non-Patent Documents 1 to 4).

However, since the above compounds have low solubility in water, it is difficult for the inhibitor contained in the preparation to dissolve in an effective amount and exhibit sufficient efficacy. In general, as a preparation that can contain a therapeutically effective amount of a poorly water-soluble drug, a capsule that encloses a liquid containing an organic solvent as a drug with improved oral absorbability has been proposed (for example, Patent Document 1). However, capsules enclosing a liquid have the problem that they tend to leak depending on the manufacturing method due to their properties. In addition, it is not preferable to use it as a vaginal insert in such a form that an irritating substance such as an organic solvent is diffused in the vagina. Furthermore, it is preferable to use solid preparations rather than liquid preparations as vaginal inserts.

Poorly water-soluble drugs tend to have poor absorbability due to their low solubility in water, resulting in low bioavailability. The above compound is also highly soluble in organic solvents such as methanol, ethanol, ethyl acetate, and acetone, but scarcely soluble in water. A drug preparation containing granules containing a combination of a drug and a solubilizing substance has been proposed, and it has been reported that a surfactant can be used as the solubilizing substance and that the granules can be coated (e.g., Patent Document 2). However, surfactants have adhesiveness and stickiness and reduce the flowability of powders and granules, so there is a problem that the amount to be added is limited when used in the preparation of pharmaceutical preparations such as tablets. Thus, it is difficult to prepare a vaginal insert that contains the above-described compound, which is a poorly water-soluble drug, in a therapeutically effective amount and that can provide sufficient absorbability into the body.

### [Citation List]

### [Patent Documents]

Patent Document 1: National Publication of International Patent Application No. 2003-508386
Patent Document 2: National Publication of International Patent Application No. 2007-517062

### [Non-Patent Documents]

Non-Patent Document 1: Ajiro et. al., Clin Cancer Res. 2018 Sep 15;24(18):4518-4528. doi: 10.1158/1078-0432.CCR-17-3119. Epub 2018 Apr 30
Non-Patent Document 2: Tanaka et. al., Antiviral Res. 2016 Sep;133:156-64. doi:10.1016/j.antiviral.2016.08.008. Epub 2016 Aug 8
Non-Patent Document 3: Okamoto et. al., Antiviral Res. 2015 Nov;123:1-4. doi:10.1016/j.antiviral.2015.08.012. Epub 2015 Aug 21
Non-Patent Document 4: Yamamoto et. al., J Clin Invest. 2014 Aug;124(8):3479-88. doi: 10.1172/JCI73805. Epub 2014 Jul 8

### [Summary of the Invention]

### [Technical Problem]

It is an object of the present invention to provide a pharmaceutical composition for the prevention and/or treatment of viral vaginal or perivaginal diseases. In particular, the object is to provide a pharmaceutical composition for preventing and/or treating cervical cancer, cervical intraepithelial neoplasia, or condyloma acuminata, which are diseases that develop in the perivaginal area caused by human papillomavirus.

### [Solution to Problem]

The present inventors have successfully produced a vaginal tablet containing the following compound with an antiviral activity against human papillomavirus and herpes simplex virus, and exhibiting good stability, dissolution, or bioabsorbability of the compound, and completed the present invention.

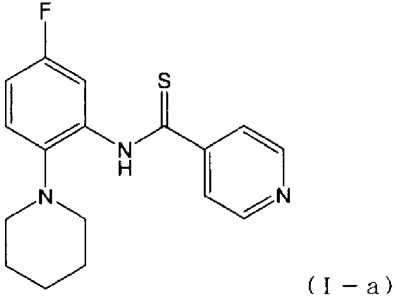

The present invention includes the following.
[1] A pharmaceutical composition for use in treating or preventing a disease caused in the vagina or perivaginal area by a pathogenic virus, the composition comprising, as an active ingredient, a compound selected from the group consisting of an aniline derivative represented by the following general formula (I): 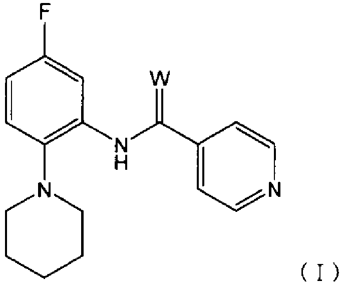
   wherein W represents S or O,
   pharmaceutically acceptable salts thereof, and hydrates thereof,
      the pharmaceutical composition being a pharmaceutical composition in the form of a vaginal insert (e.g., a vaginal tablet, a vaginal capsule or a vaginal suppository) containing a granular preparation,
      the granular preparation comprising
      core particles containing the compound, needle-like and/or approximately column-like-shaped crystalline cellulose, a pharmaceutically acceptable approximately spherical shaped additive and a nonionic surfactant,
      and a coating layer coating the core particles,
      being characterized in that, in the core particles, voids are present between the crystalline cellulose and the additive.
[2] The pharmaceutical composition for use according to [1],
   wherein the compound is a compound selected from the group consisting of an aniline derivative represented by the following formula (I-a): 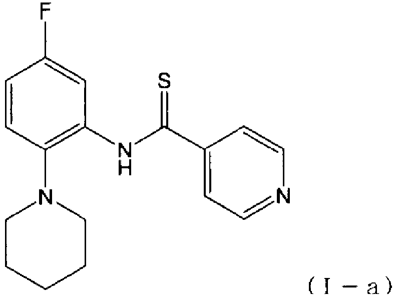
   , pharmaceutically acceptable salts thereof, and hydrates thereof.
[3] The pharmaceutical composition for use according to [1] or [2], wherein at least part of the compound and at least part of the nonionic surfactant are retained in the voids of the core particles.
[4] The pharmaceutical composition for use according to any one of [1] to [3], wherein the nonionic surfactant is contained in an amount of 2 times or less compared to the compound.
[5] The pharmaceutical composition for use according to any one of [1] to [3], wherein the nonionic surfactant is contained in an amount of 1.5 times or less compared to the compound.
[6] The pharmaceutical composition for use according to any one of [1] to [5], wherein the nonionic surfactant is contained in an amount of 1.5% by weight or more and less than 10% by weight with respect to the total amount of the pharmaceutical composition.
[7] The pharmaceutical composition for use according to any one of [1] to [5], wherein the nonionic surfactant is contained in an amount of 7 to 8% by weight with respect to the total amount of the pharmaceutical composition.
[8] The pharmaceutical composition for use according to any one of [1] to [3], wherein the nonionic surfactant is contained in an amount of 1.5% by weight or more and less than 10% by weight with respect to the total amount of the pharmaceutical composition and in an amount of 1.5 times or less compared to the compound.
[9] The pharmaceutical composition for use according to any one of [1] to [8], wherein the nonionic surfactant is polysorbate.
[10] The pharmaceutical composition for use according to any one of [1] to [9], wherein the pathogenic virus is human papillomavirus or herpes simplex virus.
[11] The pharmaceutical composition for use according to any one of [1] to [9], wherein the disease is cervical cancer or cervical intraepithelial neoplasia.
[12] The pharmaceutical composition for use according to [11],
   wherein the disease is at least one disease selected from the group consisting of diseases diagnosed as LSIL, ASC-US, ASC-H, or HSIL according to the Bethesda classification, and diseases diagnosed as low-grade dysplastic cervical intraepithelial neoplasia (CIN1), moderate dysplastic cervical intraepithelial neoplasia (CIN2) or high-grade dysplastic cervical intraepithelial neoplasia (CIN3).
[13] The pharmaceutical composition for use according to any one of [1] to [9], wherein the disease is condyloma acuminata.
[14] The pharmaceutical composition for use according to any one of [1] to [13], wherein the vaginal insert is in the form of a vaginal tablet.
[15] The pharmaceutical composition for use according to [14],
   wherein the vaginal tablet contains the compound in a dose of 10 mg to 50 mg.
[16]. The pharmaceutical composition for use according to any one of [1] to [15], wherein the compound is administered to the patient in a dose of 10 mg to 50 mg per day.
[17] The pharmaceutical composition for use according to any one of [1] to [16], wherein the compound is administered to the patient at a dose of 10 mg to 50 mg per day for 1 week to 5 weeks.
   The present disclosure includes the following:
[18] A method of producing a pharmaceutical composition for treating or preventing a disease caused in the vagina or perivaginal area by a pathogenic virus, the composition comprising, as an active ingredient, a compound selected from the group consisting of an aniline derivative represented by the following general formula (I) 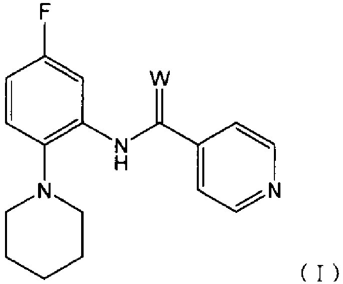
   wherein W represents S or O,
   pharmaceutically acceptable salts thereof, and hydrates thereof,
      the method comprising the following steps of:
      (a) mixing core particle raw materials containing needle-like and/or approximately column-like-shaped crystalline cellulose and a pharmaceutically acceptable approximately spherical shaped additive to obtain a core particle mixture;
      (b) dissolving or suspending the compound in a mixture of a nonionic surfactant and a solvent to obtain a mixed solution;
      (c) contacting the core particle mixture obtained in the step (a) with the mixed solution obtained in the step (b) to obtain core particles containing the compound, the needle-like and/or approximately column-like-shaped crystalline cellulose, the approximately spherical shaped additive and the nonionic surfactant;
      (d) coating the core particles obtained in the step (c) to obtain a granular preparation comprising the core particles and a coating layer coating the particles in which voids are formed between the crystalline cellulose and the approximately spherical shaped additive, and
      (e) processing the resulting granular preparation to obtain a vaginal insert in the form of a vaginal tablet, a vaginal suppository or a vaginal capsule.
[19] The production method according to [18], wherein the compound is a compound selected from the group consisting of an aniline derivative represented by the following formula (I-a): 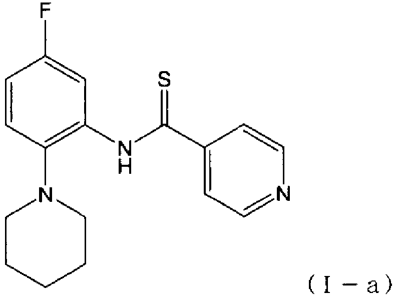 , pharmaceutically acceptable salts thereof, and hydrates thereof.
[20] The production method according to [18] or [19], wherein at least part of the compound and at least part of the nonionic surfactant are retained in voids formed in the core particles.
[21] The production method according to any one of [18] to [20], wherein the step (e) is a step of tableting the granular preparation to obtain a vaginal tablet.
[22] The production method according to any one of [18] to [21], wherein in the step (b), the nonionic surfactant is mixed in an amount of 2 times or less compared to the compound.
[23] The production method according to any one of [18] to [21], wherein in the step (b), the nonionic surfactant is mixed in an amount of 1.5 times or less compared to the compound.
[24] The production method according to any one of [18] to [23], wherein the nonionic surfactant is contained in an amount of 1.5% by weight or more and less than 10% by weight with respect to the total amount of the pharmaceutical composition.
[25] The production method according to any one of [18] to [23], wherein the nonionic surfactant is contained in an amount of 7 to 8% by weight with respect to the total amount of the pharmaceutical composition.
[26] The production method according to any one of [18] to [25], wherein the nonionic surfactant is polysorbate.
[27] The production method according to any one of [18] to [26], wherein the pathogenic virus is human papillomavirus or herpes simplex virus.
[28] The production method according to any one of [18] to [26], wherein the disease is cervical cancer or cervical intraepithelial neoplasia.
[29] The production method according to any one of [18] to [26], wherein the disease is condyloma acuminata.

### [Advantageous Effect of the Invention]

In the pharmaceutical composition of the present invention, which is a vaginal insert, a sufficient dose of the active ingredient of the present invention can be contained therein, and even in such a case, the compound as an active ingredient is stably retained, and further, when inserted into the vagina, the dissolution of the active ingredient is performed well. Because of such properties, the pharmaceutical composition of the present invention exhibits good bioabsorbability.

### [Brief Description of Drawings]

FIG. 1 is a view schematically showing the internal structure of a granular preparation contained in the pharmaceutical composition of the present invention. An active ingredient, a needle-like and/or approximately column-like-shaped core particle component 1 and an approximately spherical shaped core particle component 2 are contained in a core particle.
FIG. 2 shows the result of the dissolution of an active ingredient from a vaginal tablet, which is the pharmaceutical composition of the present invention.
FIG. 3 shows the result of the dissolution of an active ingredient from a vaginal tablet, which is the pharmaceutical composition of the present invention.
FIG. 4 shows the result of the stability of an active ingredient in a vaginal tablet, which is the pharmaceutical composition of the present invention.
FIG. 5 shows the result of the stability of an active ingredient of the pharmaceutical composition of the present invention to a surfactant.
FIG. 6 shows the result of singly administering a vaginal tablet, which is the pharmaceutical composition of the present invention, to rats and its transfer to plasma and each tissue.
FIG. 7 shows the result of repeatedly administering a vaginal tablet, which is the pharmaceutical composition of the present invention, to rats and its transfer to plasma and each tissue.
FIG. 8 shows the result of administering a vaginal tablet, which is the pharmaceutical composition of the present invention, to humans and its transfer to the blood.

### [Description of Embodiments]

Hereinafter, the present invention will be illustrated and described in detail with reference to the exemplary embodiments, along with the preferred methods and materials which can be used in practice of the present invention. Unless otherwise specified in the sentences, any technical terms and scientific terms used in the present specification have the same meaning as those generally understood by those of ordinary skill in the art to which the present invention belongs.

In the present specification, when the expression "X to Y" is used to indicate a numeral range, it means that X and Y as endpoints are included in the numeral range. In the present specification, "about" is used to mean that ±10% is allowed.

The pharmaceutical composition of the present invention can be used to treat or prevent diseases caused in the vagina or perivaginal area by pathogenic viruses. The pathogenic virus is a virus that causes a disease in the vagina or perivaginal area, and includes, for example, human papillomavirus and herpes simplex virus.

In the present specification, in the vagina or perivaginal area is used in the sense of including the vagina, vaginal cavity and opening, uterus, uterine corpus, endometrium and cervix, vulva (labia major, labia minor, perineum, and urethral meatus).

Diseases caused in the vagina or perivaginal area include, but are not limited to, cervical cancer, diseases diagnosed as LSIL, ASC-US, ASC-H, or HSIL according to the Bethesda classification, low-grade dysplasia cervical intraepithelial neoplasia (CIN1), moderate dysplasia cervical intraepithelial neoplasia (CIN2), high-grade dysplasia cervical intraepithelial neoplasia (CIN3), condyloma acuminata, and genital herpes. Generally, diagnosis of LSIL, ASC-US, ASC-H, or HSIL according to the Bethesda classification is made by vaginal cytology, and diagnosis of CIN1 to CIN3 is made by vaginal histology.

### (Active ingredient of the present invention)

The active ingredient of the pharmaceutical composition of the present invention is an aniline derivative represented by the following general formula (I): 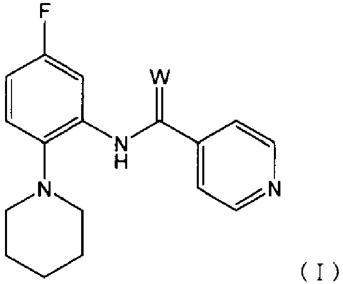
wherein W represents S or O,
or a pharmaceutically acceptable salt thereof, or a hydrate thereof (in the present specification, it may be referred to as "active ingredient of the pharmaceutical composition of the present invention", "active ingredient of the present invention", "compound of the pharmaceutical composition of the present invention" or "compound used in the present invention", and when it is clear from the context before and after, it may be simply referred to as "active ingredient".).

The drug represented by the general formula (I) includes, specifically, N-[5-fluoro-2-(1-piperidinyl)phenyl] isonicotinthioamide represented by the following formula (I-a).

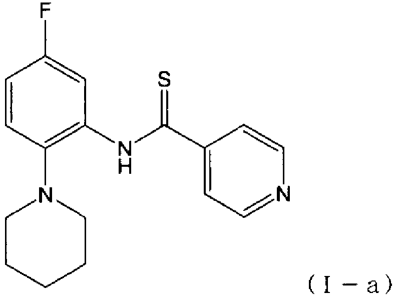

The active ingredient of the present invention exhibits an antiviral activity against pathogenic viruses. Viruses to which the active ingredient of the present invention exhibits an antiviral activity include, but are not limited to, human papillomavirus and herpes simplex virus. Among human papillomaviruses, HPV 16, 18, etc. are known as causative viruses of cervical cancer, and HPV 6, 11, etc. are known as causative viruses of condyloma acuminata, and the active ingredient of the present invention exhibits an effective antiviral activity against these viruses (e.g., Non-Patent Documents 1-4).

The pharmaceutical composition of the present invention may contain other drugs in addition to the above active ingredients. Other agents include, for example, antiviral agents, anti-inflammatory agents, antibacterial agents, antifungal agents, and the like. Antiviral agents include, for example, acyclovir. Anti-inflammatory agents include, for example, non-steroidal anti-inflammatory drugs (NSAIDs). Antibacterial agents include, for example, metronidazole and clindamycin. Antifungal agents include, for example, fluconazole and itraconazole.

The amount of the above-described active ingredient in the pharmaceutical composition of the present invention is not particularly limited as long as the pharmaceutical composition of the present invention exhibits a desired effect, and for the treatment and/or prevention of diseases caused by human papillomavirus, for example, the lower limit per tablet can be 10 mg, 20 mg, 30 mg, 40 mg, or 50 mg, and the upper limit thereof can be 1000 mg, 800 mg, 600 mg, 500 mg, 400 mg, 300 mg, 200 mg or 100 mg, and for the treatment and/or prevention of diseases caused by herpes simplex virus, for example, the lower limit per tablet can be 2 mg, 5 mg, or 10 mg, and the upper limit thereof can be 200 mg, 100 mg, or 80 mg.

### (Nonionic surfactant)

The pharmaceutical composition of the present invention contains a nonionic surfactant. The nonionic surfactant is not particularly limited as long as it is pharmaceutically acceptable, and as long as it does not have a non-negligible effect on the stability and dissolution of the active ingredient contained in the pharmaceutical composition of the present invention. It includes, for example, alkylpolyoxyethylene ethers, polyoxyethylene fatty acid esters, sorbitan fatty acid esters, sucrose fatty acid esters, polyglycerin fatty acid esters, polyoxyethylene sorbitan fatty acid esters, and the like, and is preferably polysorbate, and more preferably polysorbate 80. These surfactants may be used singly or in combination of two or more. These surfactants may be dissolved in, for example, water, alcohol, and the like, in the process of producing the pharmaceutical composition of the present invention so as to be included in the pharmaceutical composition of the present invention.

The amount of the nonionic surfactant in the pharmaceutical composition of the present invention is not particularly limited as long as it does not interfere with the object of the present invention, which is the use as a vaginal insert exhibiting an antiviral activity, and it is 1.5% by weight or more and less than 10% by weight with respect to the total amount of the pharmaceutical composition. If the content of the surfactant is more than 10% by weight, moldability and granulation property will be poor, making it difficult to produce it as a vaginal insert (in the form of vaginal tablet, vaginal capsule, or vaginal suppository). On the other hand, if the content of the surfactant is less than 1.5% by weight, the dissolution of the active ingredient from the pharmaceutical composition containing the desired therapeutically effective amount of the active ingredient may be insufficient. The content of the surfactant should be determined in consideration also of the amount of the active ingredient of the present invention contained in the pharmaceutical composition, and its lower limit can be 1.5% by weight, 2.0% by weight, 2.5% by weight, 3.0% by weight, 3.5% by weight, 4.0% by weight, 4.5% by weight, 5.0% by weight, 5.5% by weight, 6 0% by weight, 6.5% by weight, or 7.0% by weight, and its upper limit can be 10.0% by weight, 9.5% by weight, 9.0% by weight, 8.5% by weight, or 8.0% by weight, with respect to the total amount of the pharmaceutical composition. The content of the surfactant is preferably 6 to 9% by weight, more preferably 7 to 9% by weight, and further preferably 7 to 8% by weight, with respect to the total amount of the pharmaceutical composition.

In the pharmaceutical composition of the present invention, the amount of the nonionic surfactant relative to the active ingredient of the present invention is not particularly limited as long as the object of the present invention is achieved, but it is contained preferably in an amount of 2 times or less compared to the active ingredient. If the amount of the surfactant is 2 times or less compared to the active ingredient, the active ingredient is stably preserved and degradation products derived from it can be ignored. Moreover, the lower limit of the amount of the surfactant relative to the active ingredient is not particularly limited as long as the active ingredient is sufficiently dissolved, but for example, it can be at least 0.4 times compared to the active ingredient. In the pharmaceutical composition of the present invention, the surfactant is contained in an amount as the lower limit of 0.4 times, 0.5 times, 0.6 times, 0.7 times, 0.8 times, 0.9 times, or 1.0 time, and in an amount as the upper limit of 2.0 times, 1.9 times, 1.8 times, 1.7 times, 1.6 times, 1.5 times, 1.4 times, or 1.3 times compared to the active ingredient. The content of the surfactant is preferably 1.0 to 1.7 times, more preferably 1.0 to 1.6 times, further preferably 1.0 to 1.5 times, still further preferably 1.0 to 1.4 times, and most preferably about 1.2 times compared to the active ingredient.

### (Pharmaceutical composition)

The pharmaceutical composition of the present invention is in the form of a vaginal insert that is inserted into the vagina. The vaginal insert includes, for example, vaginal tablets, vaginal capsules and vaginal suppositories, preferably vaginal tablets. The vaginal insert, which is the pharmaceutical composition of the present invention, contains a granular preparation having core particles and a coating layer that coats the core particles. The core particles contain the above-described active ingredient, needle-like and/or approximately column-like-shaped crystalline cellulose, a pharmaceutically acceptable approximately spherical shaped additive, and a nonionic surfactant. The granular preparation having core particles and a coating layer coating the core particles used in the present invention (in the present specification, it may be referred to as "the granular preparation used in the present invention" or "the granular preparation of the present invention", and when it is clear from the context before and after, it may be simply referred to as "granular preparation") has good flowability. Therefore, the granular preparation can be tableted or molded into a vaginal tablet, encapsulated to give a vaginal capsule, or tableted or molded into a vaginal suppository. In preparing the vaginal insert of the present invention, molding is preferably compression molding, and encapsulation is preferably compression encapsulation.

### (Needle-like and/or approximately column-like-shaped crystalline cellulose)

The needle-like and/or approximately column-like-shaped crystalline cellulose contained in the pharmaceutical composition of the present invention (in the present specification, sometimes simply referred to as "needle-like crystalline cellulose") has a needle-like or approximately column-like shape. The needle-like crystalline cellulose is crystalline cellulose that is used as an additive in the preparation of pharmaceutical preparations, and having a needle-like or approximately column-like shape. By producing the pharmaceutical composition of the present invention with use of, as a raw material, a pharmaceutically acceptable crystalline cellulose mainly containing crystals having a needle-like or approximately column-like shape, needle-like crystalline cellulose can be contained in the pharmaceutical composition of the present invention, more specifically in the granular preparation of the present invention contained in the pharmaceutical composition. The crystalline cellulose used as a raw material may be one that can provide a sufficient proportion of needle-like and/or approximately column-like crystals to achieve the effect of the present invention when processed into the pharmaceutical composition of the present invention. In the crystalline cellulose raw materials that provide the needle-like crystalline cellulose contained in the pharmaceutical composition of the present invention, the proportion of needle-like and/or approximately column-like-shaped crystals is not particularly limited, but its lower limit is, for example, 60%, 65%, 70%, 75% or 80%, and its upper limit is, for example, 100%, 98% or 95%, with respect to the total crystal (particle) number. The number of needle-like and/or approximately column-like-shaped crystals (particles) is preferably 60 to 100%, more preferably 70 to 100%, and further preferably 80 to 100% of the total. In the present specification, the term "needle-like or approximately column-like crystalline cellulose" refers to crystalline cellulose in which there is a remarkable difference between the vertical and horizontal lengths in the longitudinal cross section of crystalline cellulose on an image (shape transferred to a plane) measured with an electron microscope. Here, the remarkable difference between the vertical and horizontal lengths can be represented by, for example, the aspect ratio.

The aspect ratio of the crystalline cellulose is not particularly limited as long as the effect of the present invention is exhibited, but its lower limit can be, for example, preferably 1.8, more preferably 2.2, and further preferably 2.5, and its upper limit can be, for example, 10, 9, or 8. The range of the average aspect ratio of the needle-like crystalline cellulose is not particularly limited, but is preferably 1.8 to 10, more preferably 2.2 to 10, and further preferably 2.5 to 10. In addition, in the crystalline cellulose raw materials that provide the needle-like crystalline cellulose contained in the pharmaceutical composition of the present invention, those having an average aspect ratio of the crystal in the range of 1.7 to 10, particularly 2.0 to 10 are preferably used in production of the pharmaceutical composition of the present invention.

The diameter of the particles contained in the crystalline cellulose raw material that provides the needle-like crystalline cellulose is not particularly limited as long as the effect of the present invention is exhibited, and the average particle size (D50) is preferably 50 to 200 µm, more preferably 60 to 150 µm, and further preferably 70 to 100 µm. The particle size and average particle size of the particles are measured, for example, using a commercially available particle size distribution meter (e.g., Mastersizer3000, produced by Spectris Co., Ltd.) by a laser diffraction method (measurement method: dry, scattering intensity: 1% or more, light scattering model: Mie scattering theory).

The content of the crystalline cellulose raw material that provides the needle-like crystalline cellulose in the entire pharmaceutical composition is not particularly limited as long as the effect of the present invention is exhibited, and its lower limit is preferably 2% by weight, more preferably 3% by weight, and further preferably 5% by weight, while its upper limit is preferably 30% by weight, more preferably 20% by weight, with respect to the total weight of the pharmaceutical composition, and the content is appropriately selected according to the content of the needle-like crystalline cellulose in the crystalline cellulose raw material, the type and amount of other additives to be added to the pharmaceutical composition, and the intended properties of the desired pharmaceutical composition. The content of the crystalline cellulose raw material that provides the needle-like crystalline cellulose with respect to the granular preparation contained in the pharmaceutical composition is not particularly limited as long as the effect of the present invention is exhibited, and its lower limit is preferably 5% by weight, more preferably 10% by weight, while its upper limit is preferably 50% by weight, more preferably 40% by weight, with respect to the total weight of the granular preparation, and the content is appropriately selected according to the content of the needle-like crystalline cellulose in the crystalline cellulose raw material, the type and amount of other additives to be added, and the intended properties of the desired pharmaceutical composition.

### (Approximately spherical shaped additive)

The pharmaceutically acceptable approximately spherical shaped additive used in the pharmaceutical composition of the present invention (herein sometimes referred to as "approximately spherical shaped additive") is an additive that can be used in the field of pharmaceuticals, and is approximately spherical shaped. Here, the approximately spherical shaped one (approximately spherical shaped additive) means that it mainly contains approximately spherical shaped particles as a component, and means, for example, an additive in which approximately spherical shaped particles are contained in a proportion of 80% or more, preferably 85 % or more, more preferably 90% or more, even further preferably 95% or more, and most preferably approximately 100% of the total. In the present specification, the term "approximately spherical shaped" refers to a shape with no significant difference between vertical and horizontal lengths on an image (shape transferred to a plane) measured by an electron microscope. Therefore, the term "approximately spherical shaped" in the present specification is used to include ellipsoidal, polyhedral (including cubic), and rounded polyhedral shapes, in addition to nearly spherical shapes. Therefore, the "approximately spherical shaped" does not necessarily mean that the image measured by an electron microscope is sphere-like itself, and may be, for example, distorted sphere-like, ellipsoidal, polyhedral (including cubic), and rounded polyhedral.

Although it is not limited to this, when considering the approximately spherical shaped in terms of aspect ratio, for example, its upper limit includes 1.7, 1.6, 1.5, 1.4, 1.3, or 1.2. Therefore, the average aspect ratio of the approximately spherical shaped additive in the core particles contained in the pharmaceutical composition of the present invention is preferably 1.0 to 1.6, more preferably 1.0 to 1.5, further preferably 1.0 to 1.3, and still further preferably 1.0 to 1.2.

The diameter of the particles contained in the raw material that provides the approximately spherical shaped additive in the core particles contained in the pharmaceutical composition of the present invention is not particularly limited as long as the effect of the present invention is exhibited, but the average particle size (D50) is preferably 1 to 300 µm, more preferably 5 to 200 µm, and further preferably 10 to 150 µm. The particle size and average particle size of the particles are measured, for example, using a commercially available particle size distribution meter (e.g., Mastersizer3000, manufactured by Spectris Co., Ltd.) by a laser diffraction method (measurement method: dry, scattering intensity: 1% or more, light scattering model: Mie scattering theory).

For example, by producing the pharmaceutical composition of the present invention using an approximately spherical shaped additive having the above average aspect ratio as a raw material, the granular preparation contained in the pharmaceutical composition of the present invention can be made to contain the approximately spherical shaped additive. The content of the approximately spherical shaped additive in the entire pharmaceutical composition is not particularly limited as long as the effect of the present invention is exhibited, but its lower limit is preferably 10% by weight, more preferably 15% by weight, while its upper limit is preferably 50% by weight, more preferably 40% by weight, with respect to the total weight of the pharmaceutical composition, and the content is appropriately selected according to the type and amount of other additives to be added to the pharmaceutical composition, and the intended properties of the desired pharmaceutical composition. For the content of the approximately spherical shaped additive in the granular preparation contained in the pharmaceutical composition, its lower limit is preferably 30% by weight, more preferably 35% by weight, while its upper limit is preferably 90% by weight, more preferably 80% by weight, with respect to the total weight of the granular preparation, and the content is appropriately selected according to the type and amount of other additives to be added, and the intended properties of the desired pharmaceutical composition.

The approximately spherical shaped additive may be used as a single component or as a combination of two or more components, preferably a combination of two or more components having different average particle sizes. When the approximately spherical shaped additive is composed of two components, it is preferable that the respective components have different average particle sizes. The lower limit of the ratio of the average particle sizes of two different components include, but not particularly limited to, for example, 1.2, 1.3, 1.4, 1.5, or 2, while its upper limit includes, but not particularly limited to, for example, 20, 15, 10, 5, 4, or 3. The ratio of the average particle sizes of two different components is not particularly limited, but is, for example, 1.2 to 20, preferably 1.3 to 10, more preferably 1.5 to 5, and further preferably 2 to 3.

The approximately spherical shaped additive is not particularly limited as long as it is a pharmaceutically acceptable additive, and includes, for example, excipients, binders, disintegrants, lubricants, and the like, and these additives can be used without limitation as long as they perform the effect of the present invention. Preferably, an additive classified as an excipient is used as the approximately spherical shaped additive in the present invention. Specific examples include, but are not limited to, sugars (including sugars, sugar hydrates, sugar alcohols, etc.), inorganic compounds, and the like.

Examples of sugars include, but are not limited to, monosaccharides such as glucose, disaccharides such as lactose and sucrose, and polysaccharides such as starch and cellulose. Starches include, for example, potato starch, wheat starch, corn starch, rice starch and the like. Corn starch is preferably used as sugar. The sugar hydrate is not particularly limited, but includes, for example, any hydrate of the sugars described above, preferably lactose hydrate. Sugar alcohols include, but are not particularly limited to, sugar alcohols derived from any sugar, preferably mannitol or sorbitol. Inorganic compounds include, but not particularly limited to, phosphates such as anhydrous calcium phosphate, and the like.

Examples of approximately spherical shaped additives preferably include disaccharides such as lactose, lactose hydrate and sucrose, etc., and corn starch and potato starch.

In the present specification, the term "aspect ratio" means the value of the ratio of the major axis to the minor axis of the particles contained in the component (major axis/minor axis) in particle image analysis using an electron microscope, and further, the term "average aspect ratio" means a value obtained by measuring the aspect ratio of 10 or more arbitrarily selected particles, excluding the aspect ratio values of the particles in the top 10% and the bottom 10% of the aspect ratio values, and averaging the aspect ratios of the remaining particles.

The total weight of the crystalline cellulose raw material that provides the needle-like crystalline cellulose and the approximately spherical shaped additive is not particularly limited as long as the effect of the present invention is exhibited, and its lower limit is, for example, 10% by weight, 20% by weight, 25% by weight, or 30% by weight, while its upper limit is, for example, 90% by weight, 80% by weight, 70% by weight, or 60% by weight, and preferably 20 to 80% by weight, and more preferably 30 to 70% by weight, with respect to total weight of the pharmaceutical composition. In addition, the ratio of the approximately spherical shaped additive to the crystalline cellulose raw material that provides the needle-like crystalline cellulose (weight ratio: approximately spherical shaped additive/crystalline cellulose raw material) is not particularly limited as long as the effect of the present invention is exhibited, but it is, for example, 1 to 10 times, and preferably 2 to 5 times.

### (Coating layer)

The coating layer can coat the core particles to prevent the surfactant or drug contained in the core particles from leaking to the surface of the granular preparation of the present invention. As a result of suppressing leakage of the surfactant by the coating layer, aggregation of the granular preparation can be suppressed, and a decrease in flowability can be suppressed, so that vaginal tablets can be produced satisfactorily.

The component that constitutes the coating layer is not particularly limited, but includes, for example, a water-soluble coating agent, and the like. The water-soluble coating agents may be used singly or in combination of two or more. According to one embodiment, the water-soluble coating agent preferably comprises at least one component selected from polyalkylene glycols and polysaccharides or derivatives thereof.

The polysaccharides or derivatives thereof are preferably cellulose derivatives, and include, for example, methylcellulose, hydroxymethylcellulose, hydroxypropylmethylcellulose, and the like. One of the cellulose derivatives may be used alone, or two or more thereof may be used in combination. Moreover, the polyalkylene glycol includes, for example, polyethylene glycol, and the like.

According to an embodiment, the coating agent may be used in combination with a plasticizer. Examples of the plasticizer include acetyl tributyl citrate, acetyl triethyl citrate, castor oil, diacetylated monoglyceride, dibutyl sebacate, sorbitol, dextrin, diethyl phthalate, glycerin, polyalkylene glycol, polyethylene glycol monoethyl ether, propylene glycol, benzyl benzoate, purified water, Sorbitol Sorbitan Solution, triacetin, tributyl citrate, triethyl citrate and chlorobutanol. Of these plasticizers, preferably a polyalkylene glycol, and more preferably polyethylene glycol (macrogol) is used. These plasticizers may be used alone or in combination of two or more.

The component constituting the coating layer may be directly used or, if necessary, dissolved in, e.g., water or an alcohol, and put in use.

The amount of the coating layer in the pharmaceutical composition of the present invention is not particularly limited as long as the granular preparation and pharmaceutical composition of the present invention exhibit the desired effect, but its lower limit is, for example, 0.001, 0.002, 0.003 or 0.005, while its upper limit is, for example, 0.1, 0.05, 0.03, or 0.02, and preferably 0.002 to 0.05, more preferably 0.005 to 0.05, and further preferably 0.005 to 0.02, in terms of the ratio to the total weight of core particles to be coated (weight ratio: weight of coating layer/total weight of core particles). In addition, for the ratio of the amount of components constituting the coating layer to the total weight of the pharmaceutical composition of the present invention (weight ratio: weight of coating layer components/total weight of pharmaceutical composition), its lower limit of is, for example, 0.0003, 0.001 or 0.002, while its upper limit is, for example, 0.05, 0.03, 0.02 or 0.01, and the ratio is preferably 0.001 to 0.03, more preferably 0.001 to 0.02, and further preferably 0.002 to 0.01.

The particle size of the granular preparation of the present invention is not particularly limited, and its lower limit is, for example, 50 µm, 60 µm, 80 µm, or 100 µm, while its upper limit is, for example, 700 µm, 500 µm, 400 µm, or 300 µm. The average particle size (D50) is, for example, 100 to 400 µm, preferably 100 to 300 µm, and more preferably 120 to 250 µm. The average particle size of the granular preparation can be measured by the same method as the measurement method described above.

Since the pharmaceutical composition of the present invention has a coating layer that coats the core particles in the granular preparation contained therein, the surfactant and drug (active ingredient) contained in the core particles are suppressed to leak from the granular preparation, and as a result, aggregation of the granular preparation can be suppressed, and it can be formulated as a vaginal insert. Although it is not limited to this, since the aggregation of the granular preparation is suppressed, molding, especially compression molding becomes possible in the production of vaginal tablets and vaginal suppositories, while encapsulation, especially compression encapsulation is facilitated in the production of vaginal capsules. The vaginal capsules include hard capsules.

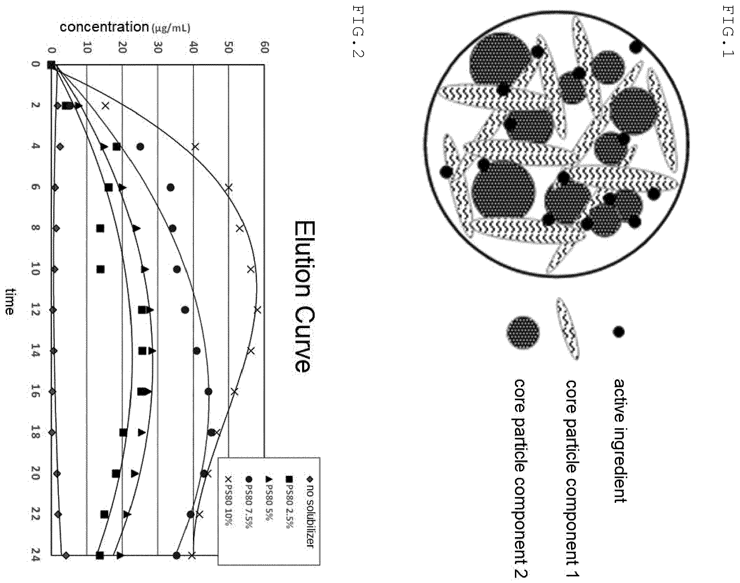

In the pharmaceutical composition of the present invention, many voids are formed between the needle-like crystalline cellulose and the approximately spherical shaped additive in the core particles in the granular preparation. FIG. 1 schematically shows their state. Therefore, the core particles can have many voids, i.e., have a good porosity, and the nonionic surfactant and the active ingredient of the present invention can enter the voids. As a result, pharmaceutical compositions are provided that retain greater amounts of the active ingredient of the present invention and the nonionic surfactant. In addition, in the pharmaceutical composition of the present invention, since a larger amount of the active ingredient and the nonionic surfactant are retained, when administered intravaginally, they can achieve well transfer to the perivaginal tissue. This is also supported by the fact that transfer to the blood is observed.

### (Other components)

The pharmaceutical composition of the present invention may contain pharmaceutically acceptable additives different from the components contained in the core particles and the components constituting the coating layer coting the particles, described above, as long as the effect of the present invention is not interfered with. The additives include, for example, excipients, disintegrants, release agents, separating agents, lubricants, binders, fluidizing agents, stabilizers, adsorbents, glossing agents, wetting agents, wetting control agents, fillers, adhesion enhancers, thickeners, desiccants, sweeteners, flavoring agents, coloring agents and the like. One component among these additives may serve two or more functions. Moreover, these additives may be used each singly or in combination of two or more.

### (Method for producing pharmaceutical composition)

The pharmaceutical composition of the present invention can be produced by the following steps.
Step a: a step of mixing core particle raw materials containing needle-like and/or approximately column-like-shaped crystalline cellulose and a pharmaceutically acceptable approximately spherical shaped additive to obtain a core particle mixture;
Step b: a step of dissolving or suspending the following compound in a mixture of a nonionic surfactant and a solvent to obtain a mixed solution,
   the compound being selected from the group consisting of an aniline derivative represented by the following general formula (I): 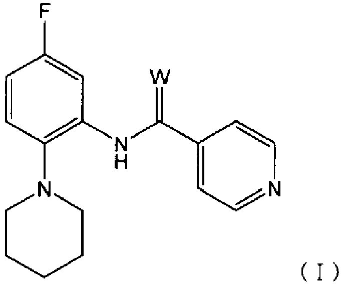
   wherein W represents S or O,
   or pharmaceutically acceptable salts thereof, and hydrates thereof,
Step c: a step of contacting the core particle mixture obtained in the step (a) with the mixed solution obtained in the step (b) to obtain core particles containing the compound, the above-described needle-like and/or approximately column-like-shaped crystalline cellulose, the approximately spherical shaped additive, and the above-described nonionic surfactant;
Step d: a step of coating the core particles obtained in the step (c) to obtain a granular preparation having the core particles and a coating layer coating the particles in which voids are formed between the crystalline cellulose and the additive, and
Step e: a step of processing the resulting granular preparation to obtain a vaginal insert in the form of a vaginal tablet, a vaginal suppository or a vaginal capsule.

As the needle-like and/or approximately column-like-shaped crystalline cellulose that can be used in step (a), those described for the pharmaceutical composition can be used. Various crystalline celluloses have been reported or are commercially available as additives that can be added to pharmaceutical compositions, and of them, those mainly containing needle-like and/or approximately column-like-shaped crystals can be used without restriction. For example, for the proportion of needle-like and/or approximately column-like-shaped crystals, its lower limit is, for example, 60%, 65%, 70%, 75%, or 80%, while its upper limit is, for example, 100%, 98%, or 95%, with respect to the total number of crystals (particles). The number of needle-like and/or approximately column-like-shaped crystals (particles) is preferably 60 to 100%, more preferably 70 to 100%, and further preferably 80 to 100% of the total. The crystalline cellulose containing a large number of needle-like and/or approximately column-like-shaped crystals includes, but not limited to, for example, CEOLUS (registered trademark) (manufactured by Asahi Kasei Corporation) and the like, which are crystalline celluloses that can be added to pharmaceuticals and contain a large number of needle-like and/or approximately column-like-shaped crystals.

As the pharmaceutically acceptable approximately spherical shaped excipient that can be used in step (a), those described for the pharmaceutical composition can be used. Examples thereof include, but are not limited to, disaccharides such as lactose, lactose hydrate and sucrose, etc., and corn starch and potato starch.

As the nonionic surfactant that can be used in step (b), those described for the pharmaceutical composition can be used. Non-limiting examples thereof include polysorbates, particularly polysorbate 80.

The solvent that can be used in step (b) includes solvents for dissolving or suspending the poorly water-soluble substance that can be used in the production of the pharmaceutical composition, capable of sufficiently dissolving or suspending the above compound. The solvent is preferably an organic solvent. In the pharmaceutical composition of the present invention, the solvent (especially, organic solvent) added in the production process volatilizes in the drying process, so it can be used without particular limitation as long as residual substances do not pose a problem. Examples thereof include, but are not limited to, methanol, ethanol, ethyl acetate, acetone, diethyl ether, and the like, preferably methanol, ethanol, or ethyl acetate, and particularly preferably methanol. Since these organic solvents volatilize during the drying step in the production method of the present invention, they do not remain in the produced pharmaceutical composition, and the pharmaceutical composition of the present invention becomes a vaginal insert that does not contain them.

In the present specification, "dissolving or suspending the compound used in the present invention" is used in the sense of including also a state in which a portion of the compound is dissolved and the other portion is suspended, in addition to a state in which the entire compound is dissolved or suspended.

In each step of the production method described above, known methods can be appropriately referred to and used. The conditions for producing the pharmaceutical composition can be appropriately adjusted by referring to known methods based on the types and amounts of components contained in the composition, the intended dosage form, and other factors. Specifically, but not limited to, the pharmaceutical composition of the present invention can be produced, for example, according to the following procedure. First, needle-like and/or approximately column-like-shaped crystalline cellulose and at least one pharmaceutically acceptable approximately spherical shaped additive are mixed in a fluidized bed granulator (for example, FD-MP-01D, manufactured by Powrex Corp.), to obtain a mixture of core particles (primary particles). On the other hand, the active ingredient of the present invention is added to a nonionic surfactant and stirred with a stirrer (e.g., NZ-1200, produced by Tokyo Rikakikai Co., Ltd.) to obtain a mixed solution (drug solution) in which the active ingredient is dissolved or suspended. Next, the resulting mixture and mixed solution are brought into contact with each other using a fluidized bed granulator to adhere the mixed solution to the core particles in the mixture to obtain core particles. Here, the mixture and the mixed solution are brought into contact with each other, for example, by spraying the mixed solution onto the mixture, by immersing the mixture in the mixed solution, or the like.

Next, after the core particles are dried as necessary, the core particles are coated with components constituting the coating layer (coating layer components). Here, the coating of the core particles is performed by, for example, a method of spraying the coating layer components onto the cores, a method of immersing the core particles in the coating layer components, or the like. Next, the particles having the core particles and the coating layer coating the core particles are dried to obtain the granular preparation of the present invention. In the method for producing the pharmaceutical composition of the present invention, "coating the core particles to obtain a granular preparation having the core particles and a coating layer coating the particles in which voids are formed between the needle-like crystalline cellulose and the approximately spherical shaped additive" means spraying coating layer components on the core particles or immersing the core particles in coating layer components, then, drying them, to obtain a granular preparation, as described above.

The granular preparation is then formulated into a vaginal insert (vaginal tablet, vaginal suppository, or vaginal capsule). Molding into a vaginal tablet can be performed, for example, by tableting. A method for tableting the granular preparation of the present invention is not particularly limited and a known method can be used. The conditions for tableting are not particularly limited and can be appropriately controlled depending on the types of core particle components, surfactant, drug and coating layer components, and the like. As a method for tableting the granular preparation, for example, a method of tableting the preparation by a tablet press such as a rotary tablet press or a single-shot tablet press. Of them, a method of tableting the preparation by a rotary tablet press is preferred. As the rotary tablet press, e.g., VIRGO 0512SS2AY manufactured by KIKUSUI is mentioned. If tablets contain pharmaceutically acceptable additive(s) other than the granular preparation of the present invention, the granular preparation of the present invention and the pharmaceutically acceptable additive(s) are first mixed and then tableted. A method for mixing the granular preparation and additives is not particularly limited and a known method can be used. As the method for mixing the granular preparation and additives, for example, a method of using a mixer such as a V-shape mixer, is mentioned. More specifically, the V-shape mixer (TCV-20) manufactured by TOKUJU CORPORATION can be used for mixing.

A method for encapsulating the granular preparation is not particularly limited and a known method can be used. More specifically, the granular preparation is encapsuled by putting the preparation in a capsule formed of a film made of, e.g., gelatin or plant-derived material. A method for putting the preparation in the capsule formed of a film is not particularly limited and a known method such as auger powder filling, die-compress system powder filling and vibration type powder filling, can be employed. For example, in the auger powder filling, granular pharmaceutical preparation supplied/dropped from a hopper into cap-shaped containers each having an open end and usually formed of a gelatin film, and directly put in capsule bodies in a predetermined amount by use of a stirring blade and rotation pressure of an auger, and then, the cap-shaped containers are coaxially joined to produce capsules.

The method of making the granular preparation of the present invention into a vaginal suppository is not particularly limited, and a known methods can be used. Bases for vaginal suppositories include, for example, but are not limited to, cocoa butter, hard fat, macrogols such as PEG 6000 (e.g., Macrogol 6000), or glycerin-gelatin mixtures. Suppositories may further include bioadhesive agents, for example, as mucoadhesive agents, to promote adherence and long-term contact of the composition to mucous membranes, for example, vaginal epithelium.

### [Examples]

The present invention will be specifically described below with reference to examples.

### (Example 1) Antiviral activity of active ingredient of the present invention

The following compound, which is one of the active ingredients of the pharmaceutical composition of the present invention, has been reported to inhibit cyclin-dependent kinase (CDK) 9 and exhibit an antiviral activity against viruses including human papillomavirus and herpes simplex virus (For example, it is reported for an anti-human papillomavirus activity in Non-Patent Document 1, an anti-HBV activity in Non-Patent Document 2, an anti-HIV activity in Non-Patent Document 3, and an anti-human herpes simplex virus activity, an anti-human cytomegalovirus activity and an anti-human adenovirus activity in Non-Patent Document 4).

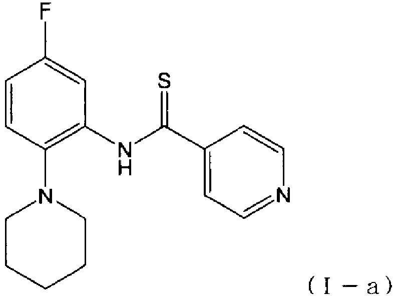

### (Anti-human papillomavirus activity)

Hela S3 cells (human cervical cancer cell line) retaining the human papillomavirus type 18 genome were seeded on a plate and cultured for 24 hours, after which the above compound was added to a final concentration of 30 µM. Cells were harvested after 3 days of culture to obtain mRNA and a cell lysate. Using the resulting mRNA as a template, real-time RT-PCR was performed using HPV18 E6 primer or GAPDH primer, and the induction magnification of the E6 gene expression rate was calculated. As a result, it was confirmed that the compound reduces the expression of the E6 gene, that is, suppresses the transcription of the E6 gene of human papillomavirus, thereby suppressing virus proliferation.

In addition, the cell lysate obtained above was developed by SDS-PAGE and Western blotting was performed using an anti-p53 antibody to evaluate p53 expression. As a result, it was confirmed that the above compound markedly increased the expression level of p53. This indicates that the compound is effective in reducing viral proteins and inhibiting degradation of cell cycle related proteins through its papillomavirus antiproliferative activity, thereby restoring normal cell function.

The compound is reported to inhibit CDK9, which is essential for human papillomavirus expression. It is believed to have an antiviral activity against other types of human papillomaviruses, such as human papillomaviruses 6, 11, etc., based on its mechanism of action.

### (Anti-herpes simplex virus activity)

Plaque assay confirmed the virus growth inhibitory effect on herpes simplex virus type 2 (HSV-2).

Vero cells (African green monkey kidney cells) were seeded on a plate and incubated overnight. The day after cell seeding, HSV-2 was infected into Vero cells, a cell culture medium containing the above compounds was added, and cultured for 24 hours. After that, a cell lysate was obtained. In order to measure the amount of HSV-2 contained in the obtained cell lysate, Vero cells were again infected with the obtained cell lysate for 1 hour and cultured in a cell culture medium containing γ-globulin for 48 hours. After culturing for 48 hours, the cells were fixed and stained with crystal violet, and the cell morphology was observed under a phase-contrast microscope, and the number of plaques derived from cell death due to HSV-2 infection was counted. As a result, it was confirmed that the above compound has an effect of suppressing the proliferation of herpes simplex virus.

### (Example 2) Preparation of pharmaceutical composition of the present invention

The granular preparation of the present invention was prepared according to the production process shown below.

### (Step 1: Core particle mixture preparation step)

Core particle raw materials including needle-like and/or approximately column-like-shaped crystalline cellulose (SEOLUS UF-702, average particle size 90 µm, produced by Asahi Kasei Corporation) and a pharmaceutically acceptable approximately spherical shaped additive (lactose hydrate (SuperTab (registered trademark), average particle size 120 µm, manufactured by DFE Pharma)) and corn starch (Cornstarch(Pharmacopoeia(JP)), average particle size 15 µm, manufactured by Nihon Shokuhin Kako Co., Ltd.) were each sieved through a 355 µm sieve and placed in a bag, and after premixing, the mixture was put into a fluidized bed granulator, to produce a core particle mixture (primary particles).

### (Step 2: Drug mixed solution preparation step)

A compound (I-a) as an active ingredient was dissolved or suspended in a mixture of a nonionic surfactant (polysorbate 80) and a solvent (ethanol) to obtain a drug mixed solution.

### (Step 3: Core particle preparation step)

Using a fluidized bed granulator, the drug mixed solution obtained in the step (2) was sprayed onto the core particle mixture (primary particles) obtained in the step (1) to obtain core particles in which the drug solution adhered to the primary particles.

### (Step 4: Final step for preparing granular preparation)

Using a fluidized bed granulator, the core particles obtained in the step (3) were sprayed with the coating layer solution and dried at 60°C to obtain a granular preparation of the present invention in which the core particles were coated with the coating layer solution.

### Next:

### (Step 5: Tableting step)

Additives (excipients, fluidizers, excipients, and lubricants in arbitrary amounts) were added to the granular preparation prepared by the above step, and these were mixed. The resulting mixture was tableted to produce a vaginal tablet, which is a vaginal insert.

### (Preparation of vaginal tablets for small animals)

According to the production method described above, pharmaceutical compositions having the formulations shown in the table below were produced as a vaginal tablet (weight of one tablet is 80 mg) for a test using small animals.

**[Table 1]**

| | | 1.25 mg tablet |
|---|---|---|
| Component | | Prescribed dose per 1 tablet (in 80 mg) |
| Granular preparation | | |
| | Compound (I-a) | 1.25 mg |
| | Lactate hydrate (SuperTab) | 11.25 mg |
| | Corn starch (cornstarch) | 5.0 mg |
| | Crystalline cellulose (CEOLUS UF-702) | 5.0 mg |
| | Polysorbate 80 | 6.0 mg |
| | Ethanol | adequate dose |
| | Hypromellose | adequate dose |
| | Macrogol 6000 | adequate dose |
| | Purified water | adequate dose |
| Additive (added before tableting) | | |
| | Lactose hydrate | adequate dose |
| | Crystalline cellulose | adequate dose |
| | Light anhydrous silicic acid | adequate dose |
| | Hydroxypropylcellulose | adequate dose |
| | Magnesium stearate | adequate dose |

### (Preparation of vaginal tablet for human administration)

According to the production method described above, pharmaceutical compositions having the formulations shown in the table below were produced as a vaginal tablet (weight of one tablet is 800 mg) for administration to humans.

**[Table 2]**

| | 10 mg tablet | 25 mg tablet | 50 mg tablet |
|---|---|---|---|
| Component | Prescribed dose per 1 tablet | Prescribed dose per 1 tablet | Prescribed dose per 1 tablet |
| | (in 800 mg) | (in 800 mg) | (in 800 mg) |
| Granular preparation | | | |
| Compound (I-a) | 10 mg | 25 mg | 50 mg |
| Lactate hydrate (SuperTab) | 115 mg | 100 mg | 75 mg |
| Corn starch (cornstarch) | 50 mg | 50 mg | 50 mg |
| Crystalline cellulose (CEOLUS UF-702) | 50 mg | 50 mg | 50 mg |
| Polysorbate 80 | 12 mg | 30 mg | 60 mg |
| Ethanol | adequate dose | adequate dose | adequate dose |
| Hypromellose | adequate dose | adequate dose | adequate dose |
| Macrogol 6000 | adequate dose | adequate dose | adequate dose |
| Purified water | adequate dose | adequate dose | adequate dose |
| Additive (added before tableting) | | | |
| Lactose hydrate | adequate dose | adequate dose | adequate dose |
| Crystalline cellulose | adequate dose | adequate dose | adequate dose |
| Light anhysrous silicic acid | adequate dose | adequate dose | adequate dose |
| Hydroxypropylcellulose | adequate dose | adequate dose | adequate dose |
| Magnesium stearate | adequate dose | adequate dose | adequate dose |

### (Example 3) Dissolution test of active ingredient in vaginal tablet

Based on the formulation of the 50 mg tablet described in Example 2, vaginal tablets (weight of one tablet is 800 mg) were produced in which the content of the compound (I-a) was 50 mg, and the content of polysorbate 80 (PS80) was 20 mg (2.5% with respect to the total amount of the vaginal tablet), 40 mg (5.0% the same standard), 60 mg (7.5% the same standard), or 80 mg (10% the same standard). As a control, a vaginal tablet containing no polysorbate 80 was also produced in the same manner.

One vaginal tablet was placed in a stainless steel tube of φ20×50 mm (about 3 mL) packed with glass beads (φ about 0.7 mm). Water was sent to the stainless steel tube at 37°C and 0.007 mL/min, sampling was performed every 120 minutes, and the dissolved compound was detected. The results are shown in FIG. 2. Dissolution of the compound could not be detected with the tablet containing no polysorbate 80, whereas with the tablet containing polysorbate 80, the dissolution increased depending on the content of polysorbate 80.

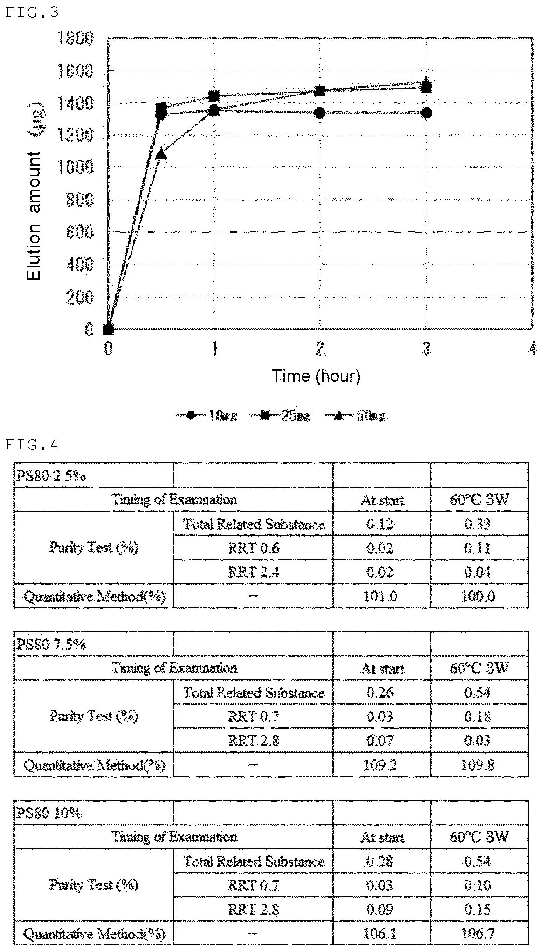

Next, the effect of the content of the compound (I-a) in the vaginal tablet on dissolution was examined. Based on the formulation of the 50 mg tablet described in Example 2, vaginal tablets (weight of one tablet is 800 mg) were produced in which the content of polysorbate 80 (PS80) was 60 mg, and the content of the compound (I-a) was 10 mg, 25 mg, or 50 mg. According to the dissolution test method of the Japanese Pharmacopoeia general test method, using 700 mL of McIlvaine buffer solution (pH 4.0) diluted as the test liquid, the test was performed at 50 rotations per minute by a paddle method. The results are shown in FIG. 3.

From the above results, it was confirmed that the compound was rapidly dissolved by combining polysorbate 80.

### (Example 4) Stability test of active ingredient in vaginal tablet

Based on the formulation of the 50 mg tablet described in Example 2, vaginal tablets (weight of one tablet is 800 mg) were produced in which the content of the compound (I-a) was 50 mg, and the content of polysorbate 80 (PS80) was 20 mg (2.5% with respect to the total amount of the vaginal tablet), 60 mg (7.5% the same standard), or 80 mg (10% the same standard). The ratio of polysorbate to the compound (I-a) in each tablet was PS80 2.5% tablet (0.4 times), PS80 7.5% tablet (1.2 times), or PS80 10% tablet (1.6 times). Each tablet was stored at 60°C and non-controlled humidity for 3 weeks, and then the degradation products were measured to examine the stability. The results are shown in FIG. 4. In the figure, RRT0.6, RRT0.7, RRT2.4 or RRT2.8 represent different degradation products, respectively. In any content of polysorbate 80, it was stable with only a slight increase in degradation products.

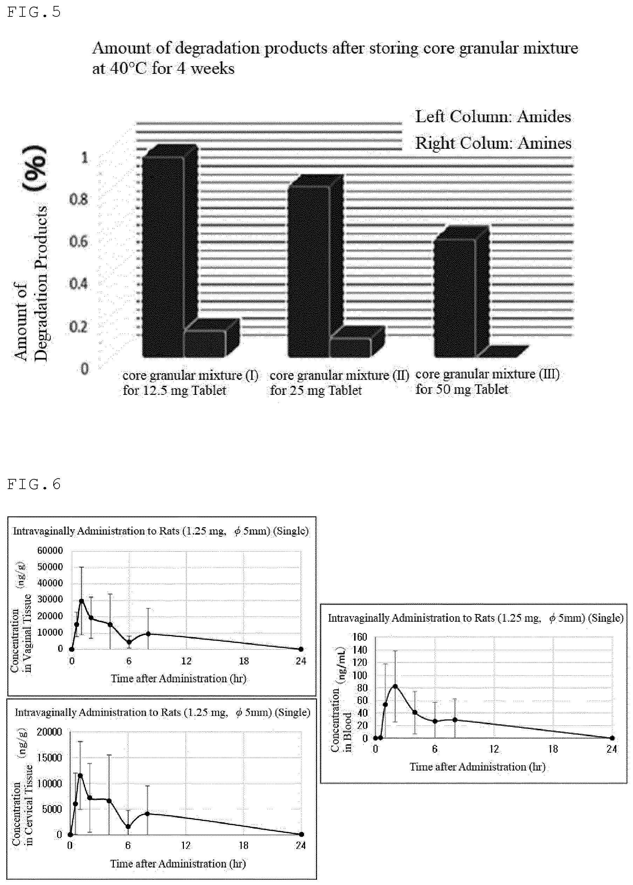

In order to examine the effect of the abundance ratio of compound (I-a) to polysorbate on stability, the compound was tested for storage stability in a state of being suspended in a polysorbate solution. Specifically, polysorbate 80 was dissolved in 2 g of ethanol and the compound was dispersed, it was dried under reduced pressure for 3 hours, then, the dried sample was allowed to stand at 40°C for 3 hours to remove the ethanol to obtain a sample. The following three samples were prepared. The compound: Polysorbate 80 (ratio of polysorbate to the compound) = (I) 12.5 mg/60 mg (4.8 times); (II) 25 mg/60 mg (2.4 times); (III) 50 mg/60 mg (1.2 times). Each sample was stored at 40°C for 4 weeks and then analyzed to detect degradation products. The results are shown in FIG. 5. A related substance (degradation product) could be significantly detected at the abundance ratio of (I) and (II).

Next, the effect of the abundance ratio of the compound (I-a) and polysorbate in the vaginal tablet on stability was examined.

Based on the formulation of the 25 mg tablet described in Example 2, vaginal tablets (weight of one tablet is 800 mg) were produced in which the content of the compound (I-a) was 25 mg, and the content of polysorbate 80 (PS80) was 30 mg (the ratio of polysorbate to the compound was 1.2 times: tablet B) or 60 mg (2.4 times the same standard: tablet A). Each tablet was subjected to an accelerated stability test (40°C ± 2°C/75% RH ± 5% RH) and the purity was measured after 1 month. The results are shown in the table below.

**[Table 3]**

| Purity test (%) | Tablet A compound: 25 mg & PS80: 60 mg | Tablet B compound: 25 mg & PS80: 30 mg |
|---|---|---|
| Total amount of related substance | 0.47% | 0.24% |

| | | |
|---|---|---|
| The above values are the averages of 3 times run. | | |

The tablet B contained significantly less related substances (degradation products) than the tablet A.

### (Example 5) Vaginal tablet administration test using rat

The vaginal tablets for small animals produced in Example 2 were administered vaginally to rats, and absorption (transfer) into plasma, cervical and vaginal tissue was detected.

The test was designed and carried out in accordance with ethical standards for animal research.

The rat was manually restrained under no anesthesia, and a vaginal tablet was placed deep in the rat's vagina (in a part near the cervix) using a jig for vaginal administration. The vaginal tablets were administered into the rat vagina singly and once daily for 5 days repeatedly, and blood, cervical and vaginal tissues were collected to analyze the concentration of the compound (I-a) in each tissue.

Each sampling was performed as follows.

### (Blood collection from the jugular vein)

Blood was collected from the jugular vein of 3 rats selected at random just before administration on days 2, 3, 4 and 5 of repeated administration.
(Whole blood collection, excision of each tissue, preparation of plasma and each tissue homogenate)

At 7 time points at 0.5, 1, 2, 4, 6, 8 and 24 hours after administration on the first day of administration and 8 time points at 0.5, 1, 2, 4, 6, 8, 24 and 72 hours after administration on the last day of repeated administration, plasma, cervical tissue and vaginal tissue were collected from 3 animals in each group and stored at -80°C. Specifically, it was carried out as follows.

Isoflurane inhalation anesthesia was performed, the abdomen was opened, and blood was collected from the abdominal aorta. Plasma was stored at -80°C until concentration determination. Immediately after blood collection, the blood was exsanguinated, the whole uterus was removed from the vaginal tissue, and the vaginal tissue washed with physiological saline from the uterine side. The surplus portion of the uterus and the vagina were cut, and the whole cervical tissue was washed again with physiological saline, water was lightly wiped off, immediately frozen with dry ice, and stored at -80°C. The uterine tissue was cut, the remaining vaginal opening was cut off, washed with physiological saline, lightly wiped to remove water, immediately frozen with dry ice, and stored at -80°C.

Five times the volume of physiological saline was added to each excised frozen tissue, and the tissue was pulverized with a beads crusher using a metal crusher to prepare a homogenate. The prepared homogenate was stored at -80°C until concentration determination.

### (Concentration determination in sample)

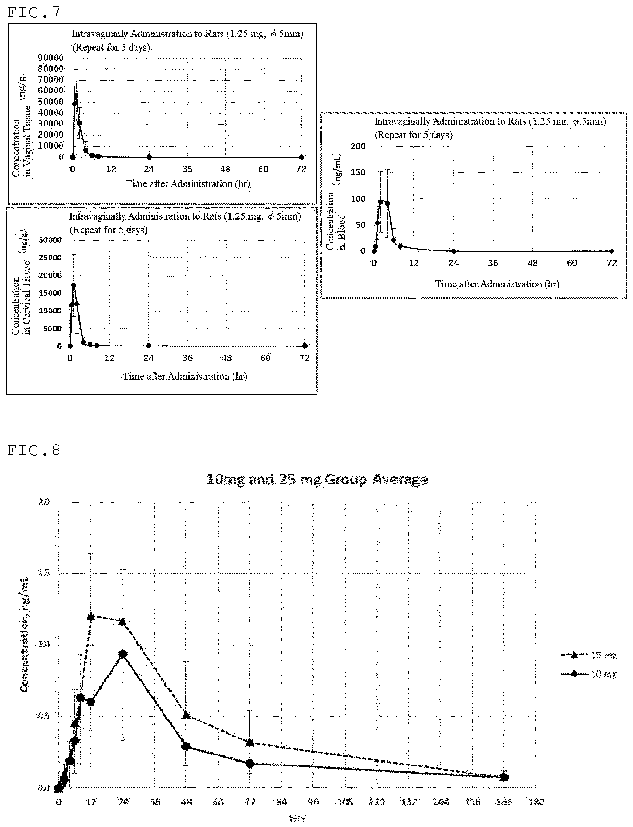

The compound concentration in each sample was quantified using LC-MS/MS. The results of single administration are shown in FIG. 6 and the results of repeated administration are shown in FIG. 7.

As a result, when the vaginal tablet of the present invention was intravaginally administered to rats, it was found to be transferred into the blood, and excellent transfer to the uterine cervix and vaginal tissue was confirmed.

### (Example 6) Test of intravaginal administration of vaginal tablet to human

Administration to humans was carried out using the human vaginal tablets (10 mg tablet and 25 mg tablet) produced in Example 2.

The vaginal tablets for humans (10 mg tablet and 25 mg tablet) produced according to Example 2 were given to a total of 12 healthy females aged 20 or more and less than 50 (6 in each dose group) and one tablet of the 10 mg tablet or the 25 mg tablet was administered by manually inserting it into the vagina. Blood was collected using a vacuum blood collection tube containing EDTA before administration, 1 hour, 2 hours, 4 hours, 6 hours, 8 hours, 12 hours, 24 hours, 48 hours, 72 hours, and 7 days after administration, and plasma was separated. The drug concentration in the separated plasma was measured by LC/MS/MS method. The results are shown in FIG. 8.

Further, the PK parameter was determined as follows. Cₘₐₓ was the maximum blood concentration of each subject, Tₘₐₓ was the time to reach the maximum blood concentration, and AUC_{0-Day8} was determined by the trapezoidal method from the change in blood concentration from before administration (0 hour on Day 1) to 7 days after administration (Day 8), and the average value and standard deviation in each dose group were calculated. The results are shown below.

**[Table 4]**

| | Cₘₐₓ ng/mL | Tₘₐₓ hrs. | AUC_{0-Day8} ng·hr/mL |
|---|---|---|---|
| 10mg group | 1.21 ± 0.41 | 18.7 ± 7.5 | 45.8 ± 15.8 |
| 25 mg group | 1.37 ± 0.46 | 18.0 ± 6.0 | 69.1 ± 29.5 |

As a result of the above, it was confirmed that the vaginal tablet of the present invention has the ability to transfer the active ingredient into the blood. In addition, since the drug was detected in the blood more than 24 hours after administration, it was thought that the active ingredient transfered to intravaginal tissue, such as the vagina and cervix, which was the administration site and target tissue, and then into the blood. Therefore, similar to Example 5, when the vaginal tablet of the present invention was administered intravaginally to humans, it was found to be transferred into the blood, and excellent transfer to the cervical and vaginal tissues was confirmed.

### [Industrial Applicability]

The pharmaceutical composition of the present invention is useful for the prevention and/or treatment of viral vaginal or perivaginal diseases.

## Claims

1. A pharmaceutical composition for use in treating or preventing a disease caused in the vagina or perivaginal area by a pathogenic virus, the composition comprising, as an active ingredient, a compound selected from the group consisting of an aniline derivative represented by the following general formula (I): 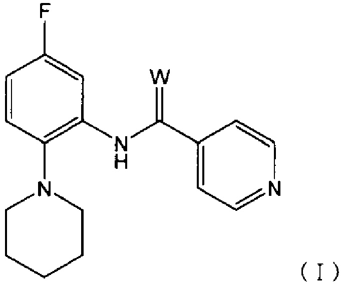
wherein W represents S or O,
pharmaceutically acceptable salts thereof, and hydrates thereof,
the pharmaceutical composition being a pharmaceutical composition in the form of a vaginal insert containing a granular preparation,
the granular preparation comprising
core particles containing the compound, needle-like and/or approximately column-like-shaped crystalline cellulose, a pharmaceutically acceptable approximately spherical shaped additive and a nonionic surfactant,
and a coating layer coating the core particles,
being **characterized in that**, in the core particles, voids are present between the crystalline cellulose and the additive.

2. The pharmaceutical composition for use according to claim 1, wherein the compound is a compound selected from the group consisting of an aniline derivative represented by the following formula (I-a): 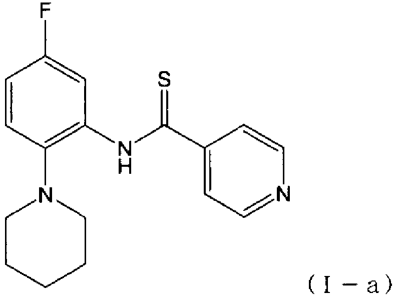 , pharmaceutically acceptable salts thereof, and hydrates thereof.

3. The pharmaceutical composition for use according to claim 1 or 2, wherein at least part of the compound and at least part of the nonionic surfactant are retained in the voids of the core particles.

4. The pharmaceutical composition for use according to any one of claims 1 to 3, wherein the nonionic surfactant is contained in an amount of 2 times or less compared to the compound, preferably 1.5 times or less.

5. The pharmaceutical composition for use according to any one of claims 1 to 4, wherein the nonionic surfactant is contained in an amount of 1.5% by weight or more and less than 10% by weight with respect to the total amount of the pharmaceutical composition, preferably 7 to 8% by weight.

6. The pharmaceutical composition for use according to any one of claims 1 to 5, wherein the nonionic surfactant is polysorbate.

7. The pharmaceutical composition for use according to any one of claims 1 to 6, wherein the pathogenic virus is human papillomavirus or herpes simplex virus.

8. The pharmaceutical composition for use according to any one of claims 1 to 6, wherein the disease is cervical cancer or cervical intraepithelial neoplasia, preferably at least one disease selected from the group consisting of diseases diagnosed as LSIL, ASC-US, ASC-H, or HSIL according to the Bethesda classification, and diseases diagnosed as low-grade dysplastic cervical intraepithelial neoplasia (CIN1), moderate dysplastic cervical intraepithelial neoplasia (CIN2) or high-grade dysplastic cervical intraepithelial neoplasia (CIN3).

9. The pharmaceutical composition for use according to any one of claims 1 to 8, wherein the vaginal insert is in the form of a vaginal tablet.

10. The pharmaceutical composition for use according to claim 9, wherein vaginal tablet contains the compound in a dose of 10 mg to 50 mg.

11. The pharmaceutical composition for use according to any one of claims 1 to 10, wherein the compound is administered to the patient in a dose of 10 mg to 50 mg per day, preferably in a dose of 10 mg to 50 mg per day for 1 week to 5 weeks.

## Patentansprüche

1. Pharmazeutische Zusammensetzung zur Verwendung bei der Behandlung oder Vorbeugung einer durch einen pathogenen Virus in der Vagina oder im perivaginalen Bereich verursachten Krankheit, wobei die Zusammensetzung als Wirkstoff eine Verbindung umfasst, die ausgewählt ist aus der Gruppe bestehend aus einem Anilinderivat, dargestellt durch die folgende allgemeine Formel (I), pharmazeutisch akzeptable Salze davon und Hydrate davon: 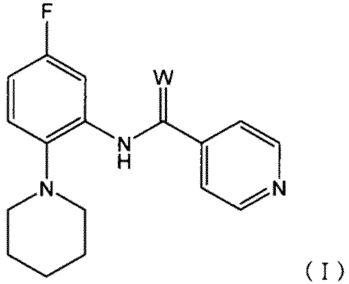
worin W S oder O darstellt,
wobei die pharmazeutische Zusammensetzung eine pharmazeutische Zusammensetzung in Form eines Vaginalinserts ist, enthaltend eine Granulatzubereitung,
wobei die Granulatzubereitung umfasst:
Kernpartikel, enthaltend die Verbindung, nadelförmige und/oder annähernd säulenförmige kristalline Cellulose, ein pharmazeutisch akzeptables annähernd kugelförmiges Additiv und ein nichtionisches Tensid,
und eine Beschichtungsschicht, die die Kernpartikel beschichtet,
**dadurch gekennzeichnet, dass** in den Kernpartikeln Hohlräume zwischen der kristallinen Cellulose und dem Additiv vorhanden sind.

2. Pharmazeutische Zusammensetzung zur Verwendung gemäß Anspruch 1, wobei die Verbindung eine Verbindung ist, die ausgewählt ist aus der Gruppe bestehend aus einem Anilinderivat, dargestellt durch die folgende allgemeine Formel (I-a), pharmazeutisch akzeptable Salze davon und Hydrate davon:

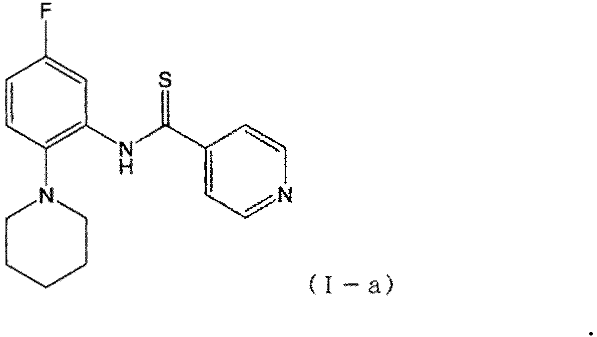

3. Pharmazeutische Zusammensetzung zur Verwendung gemäß Anspruch 1 oder 2, wobei zumindest ein Teil der Verbindung und zumindest ein Teil des nichtionischen Tensids in den Hohlräumen der Kernpartikel verbleiben.

4. Pharmazeutische Zusammensetzung zur Verwendung gemäß mindestens einem der Ansprüche 1 bis 3, wobei das nichtionische Tensid in einer Menge von 2-mal oder weniger im Vergleich zur Verbindung enthalten ist, vorzugsweise 1,5-mal oder weniger.

5. Pharmazeutische Zusammensetzung zur Verwendung gemäß mindestens einem der Ansprüche 1 bis 4, wobei das nichtionische Tensid in einer Menge von 1,5 Gew.-% oder mehr und weniger als 10 Gew.-%, bezogen auf die Gesamtmenge der pharmazeutischen Zusammensetzung, enthalten ist, vorzugsweise 7 bis 8 Gew.-%.

6. Pharmazeutische Zusammensetzung zur Verwendung gemäß mindestens einem der Ansprüche 1 bis 5, wobei das nichtionische Tensid Polysorbat ist.

7. Pharmazeutische Zusammensetzung zur Verwendung gemäß mindestens einem der Ansprüche 1 bis 6, wobei das pathogene Virus das humane Papillomavirus oder das Herpes-simplex-Virus ist.

8. Pharmazeutische Zusammensetzung zur Verwendung gemäß mindestens einem der Ansprüche 1 bis 6, wobei die Krankheit Gebärmutterhalskrebs oder zervikale intraepitheliale Neoplasie ist, vorzugsweise mindestens eine Krankheit ist, die ausgewählt ist aus der Gruppe bestehend aus Krankheiten, die gemäß der Bethesda-Klassifikation als LSIL, ASC-US, ASC-H oder HSIL diagnostiziert sind, und Krankheiten, die als niedriggradige dysplastische zervikale intraepitheliale Neoplasie (CIN1), mäßiggradige dysplastische zervikale intraepitheliale Neoplasie (CIN2) oder hochgradige dysplastische zervikale intraepitheliale Neoplasie (CIN3) diagnostiziert sind.

9. Pharmazeutische Zusammensetzung zur Verwendung gemäß mindestens einem der Ansprüche 1 bis 8, wobei das Vaginalinsert in Form einer Vaginaltablette ist.

10. Pharmazeutische Zusammensetzung zur Verwendung gemäß Anspruch 9, wobei die Vaginaltablette die Verbindung in einer Dosis von 10 mg bis 50 mg enthält.

11. Pharmazeutische Zusammensetzung zur Verwendung gemäß mindestens einem der Ansprüche 1 bis 10, wobei die Verbindung dem Patienten in einer Dosis von 10 mg bis 50 mg pro Tag verabreicht wird, vorzugsweise in einer Dosis von 10 mg bis 50 mg pro Tag für 1 Woche bis 5 Wochen.

## Revendications

1. Composition pharmaceutique destinée à être utilisée dans le traitement ou la prévention d'une maladie causée dans le vagin ou la région périvaginale par un virus pathogène, la composition comprenant, en tant qu'ingrédient actif, un composé choisi dans le groupe constitué par un dérivé d'aniline représenté par la formule générale suivante (I) : 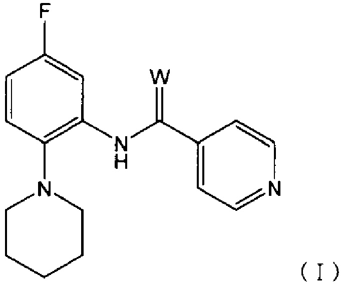
où W représente S ou O,
leurs sels pharmaceutiquement acceptables et leurs hydrates,
la composition pharmaceutique étant une composition pharmaceutique sous forme d'insert vaginal contenant une préparation granulaire,
la préparation granulaire comprenant
des particules à noyau contenant le composé, de la cellulose cristalline en forme d'aiguille et/ou approximativement en forme de colonne, un additif pharmaceutiquement acceptable de forme approximativement sphérique et un agent tensioactif non ionique,
et une couche d'enrobage recouvrant les particules à noyau,
étant **caractérisée en ce que**, dans les particules à noyau, des vides sont présents entre la cellulose cristalline et l'additif.

2. Composition pharmaceutique destinée à être utilisée selon la revendication 1, dans laquelle le composé est un composé choisi dans le groupe constitué par un dérivé d'aniline représenté par la formule suivante (I-a) : 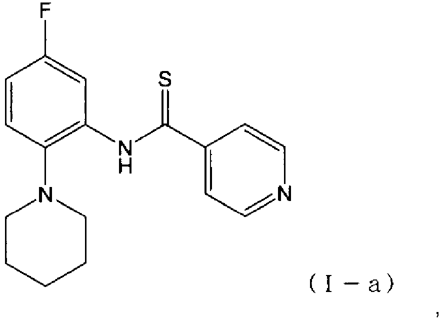 leurs sels pharmaceutiquement acceptables, et leurs hydrates.

3. Composition pharmaceutique destinée à être utilisée selon la revendication 1 ou 2, dans laquelle au moins une partie du composé et au moins une partie de l'agent tensioactif non ionique sont retenus dans les vides des particules à noyau.

4. Composition pharmaceutique destinée à être utilisée selon l'une quelconque des revendications 1 à 3, dans laquelle l'agent tensioactif non ionique est contenu dans une quantité égale ou inférieure à 2 fois celle du composé, de préférence égale ou inférieure à 1,5 fois.

5. Composition pharmaceutique destinée à être utilisée selon l'une quelconque des revendications 1 à 4, dans laquelle l'agent tensioactif non ionique est contenu à raison de 1,5 % en poids ou plus et de moins de 10 % en poids par rapport à la quantité totale de la composition pharmaceutique, de préférence de 7 à 8 % en poids.

6. Composition pharmaceutique destinée à être utilisée selon l'une quelconque des revendications 1 à 5, dans laquelle l'agent tensioactif non ionique est un polysorbate.

7. Composition pharmaceutique destinée à être utilisée selon l'une quelconque des revendications 1 à 6, dans laquelle le virus pathogène est le papillomavirus humain ou le virus de l'herpès simplex.

8. Composition pharmaceutique destinée à être utilisée selon l'une quelconque des revendications 1 à 6, dans laquelle la maladie est un cancer du col de l'utérus ou une néoplasie intraépithéliale du col de l'utérus, de préférence au moins une maladie choisie dans le groupe constitué par des maladies diagnostiquées comme LSIL, ASC-US, ASC-H, ou HSIL selon la classification de Bethesda, et des maladies diagnostiquées comme néoplasie cervicale intraépithéliale dysplasique de bas grade (CIN1), néoplasie cervicale intraépithéliale dysplasique modérée (CIN2) ou néoplasie cervicale intraépithéliale dysplasique de haut grade (CIN3).

9. Composition pharmaceutique destinée à être utilisée selon l'une quelconque des revendications 1 à 8, dans laquelle l'insert vaginal se présente sous la forme d'un comprimé gynécologique.

10. Composition pharmaceutique destinée à être utilisée selon la revendication 9, dans laquelle le comprimé gynécologique contient le composé dans une dose de 10 mg à 50 mg.

11. Composition pharmaceutique destinée à être utilisée selon l'une quelconque des revendications 1 à 10, dans laquelle le composé est administré au patient à raison de 10 mg à 50 mg par jour, de préférence à raison de 10 mg à 50 mg par jour pendant 1 semaine à 5 semaines.
